# EUROPEAN PATENT APPLICATION

(11) **EP 2 243 767 A1**
(43) Date of publication of application: **27.10.2010**
(21) Application number: 09158319.5
(22) Date of filing: 21.04.2009
(51) Int. Cl.: C07C 227/16

(54) **Process for the iodination of aromatic compounds**

(71) Applicant: Bracco Imaging S.p.A, 20134 Milano (IT); Politecnico di Milano, 20133 Milano (IT)
(72) Inventor: CITTERIO, Attilio, 20133, Milano (IT); LATTUADA, Luciano, 1-10010, Ivrea (IT); LEONARDI, Gabriella, 20133, Milano (IT); UGGERI, Fulvio, 1-10010, Ivrea (IT); VIGNALE, Evelin, 14057, Isola d'Asti (IT); VISIGALLI, Massimo, 1-10010, Ivrea (IT)
(74) Representative: Macchetta, Francesco

(57) **Abstract**

The present invention relates to a process for the preparation of iodinated anilines; in particular, it relates to a process including the direct iodination of 3,5-disubstituted anilines to the corresponding 3,5-disubstituted-2,4,6-triiodoanilines, which are useful intermediates for the synthesis of x-ray contrast media, and to the preparation of the contrast media themselves.

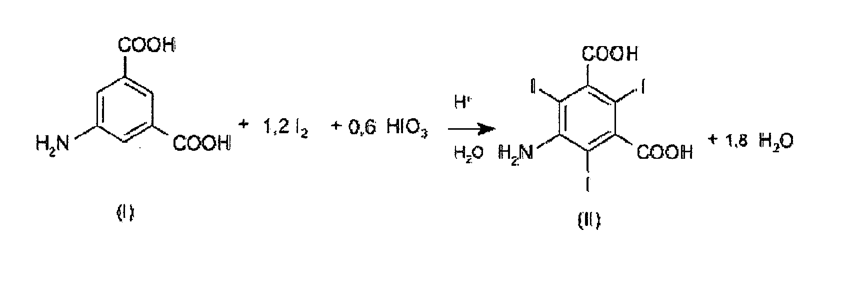

## Description

The present invention relates to a process for the iodination of aromatic compounds so as to prepare poly-iodinated aromatic derivatives. In particular, it relates to a process including the direct iodination of 3,5-disubstituted anilines to the corresponding 3,5-disubstituted-2,4,6-triiodoanilines, which are useful intermediates for the synthesis of x-ray contrast media, and to the preparation of the contrast media themselves.

### Background

Iodinated contrast media are well-known compounds widely used in x-ray imaging diagnostic techniques. Suitable examples of the said compounds include, for instance, diatrizoate, iothalamate, ioxithalamate, metrizoate, iohexol, iomeprol, iopamidol, iopentol, iopromide, ioversol, ioxilan, iodixanol, iosarcol, iogulamide, ioglunide, iogluamide, acetrizoate, iodamide, iocetamide and metrizamide, which are all monomeric; while, for example, ioxaglate, iotrolan, iotasul, iodipamide, iocarmate, iodoxamate, iotroxate, iotrolan, and the like, are dimers. Other examples of iodinated contrast agents are described, for instance, in WO 94/14478 (Bracco).

As a common feature, their chemical structure shares a triiodinated aromatic nucleus which provides the enhanced contrast effect.

The said compounds may be prepared by a variety of routes, some of which comprise the aromatic iodination of given substrates, for instance of suitable 3,5-disubstituted phenols, which undergo triiodination on the available 2, 4 and 6 positions, thus leading to the corresponding 3,5-disubstituted-2,4,6-triiodophenols. These latter, in turn, may be further converted and processed through the so-called Smile's rearrangement, to the expected final compounds. For a general reference to the above synthetic route and Smile's rearrangement see, for instance, WO 88/09328, WO 97/05097 and WO 00/32561 (Bracco). Moreover, the aromatic iodination may be also performed on suitable anilines, so as to provide the corresponding 2,4,6-triiodoaniline derivatives, to be further converted and processed to the final compounds.

The iodination reaction on both the phenol and aniline rings normally occur at the ortho and para free positions, i.e. at the 2, 4 and 6 positions, according to the well-known electrophilic substitution mechanism, thus leading to the triiodinated ring.

The iodination step may be performed utilizing different procedures. For instance, according to the known processes for the preparation of the above radiographic contrast agents, the iodination of the aromatic ring may be carried out by using solutions of iodine chloride (IC1) in concentrated hydrochloric acid (HC1) or, alternatively, by means of analogous iodinating agents such as, for instance, KICl2 or NaICl2 in aqueous solution; see, for a general reference, WO 92/14695 (Guerbet) or US 5,013,865 (Mallinckrodt).

The above methods suffer from major drawbacks mainly due to the corrosive properties of the iodinating agents and to their limited storage life. As such, particularly when considering large amounts of reactants and substrates to be employed, for instance on an industrial scale, the need of preparing the iodinating agent before usage and its storage may become particularly troublesome.

In addition, the presence of chlorine atoms within the iodinating agents themselves may lead to side-reactions and, thus, to the undesired formation of chlorine side-products, which may affect reaction yields and purity of the final compounds.

In this regard, it is worth noting that within the processes for the preparation of diagnostic contrast agents, and among them of 2,4,6-triiodobenzene derivatives, the need of collecting reaction intermediates and final compounds with a high degree of purity is of utmost importance so as to optimize, to a very significant extent, the purification steps required for the product intended for the administration, that has to be in compliance with the strict purity profile and limits imposed by the Pharmacopoeia for the product intended for the administration.

In addition, it is an increasingly recognized need to have manufacturing processes consenting to combine low production costs, process efficiency and optimal environmental impact.

Thus, attempts have been devoted to address iodination methods comprising the use of iodinating agents alternative to iodine chloride or derivatives thereof. Among them are, for instance, the electrochemical iodination processes of 3,5-disubstituted anilines or of given 3,5-disubstituted phenols, as disclosed, respectively, in WO 96/37461 and in co-pending, still unpublished, European patent application No. 08151661.9.

Beside the above electrochemical synthetic approaches, the iodination of given phenol derivatives, referred to as *ortho*-hydroxy substituted aromatic carbonyl compounds, in the presence of molecular iodine suitably activated with a strong oxidizing agent, including iodic acid, has been described by Patil et al. in Tetrahedron Letters 46 (2005) 7179-7181; and in ARKIVOC 2006, 104-108. For contrast, the possibility of exploiting that same synthetic approach, namely the combined use of molecular iodine and an oxidizing agent, to iodinate or poly-iodinate aniline or aniline derivatives is not referred in the cited art.

A method for producing monoiodinated aniline derivatives, namely 2-amino-5-iodobenzoic acid, in which 2-aminobenzoic acid, solubilised in acetic acid, is iodinated with iodine in the presence of an oxidizing agent, especially hydrogen peroxide, is disclosed in US 2007/0219396. This reference is silent on the possibility of exploiting the provided process to produce poly-iodinated and, especially, triiodinated aniline derivatives.

In this respect, it is worth noting that the use of strong oxidizing agents with aniline or even halogenated anilines is known to lead to the formation of mixtures of colored by-products, mainly azo-compounds deriving from oxidative coupling reactions involving the aromatic amino group (see, for instance, Erich Baer and Anthony L. Tosoni, J. Am. Chem. Soc., 1956, 78 (12), 2857-2858).

In spite of this major drawback, we have now unexpectedly found that the triiodination of suitable 3,5-disubstituted anilines can be advantageously carried out in high yields and purity by using a iodinating system comprising molecular iodine and an oxidizing agent.

### Object of the invention

The present invention thus provides a process for the triiodination of 3,5-disubstituted anilines with suitably activated iodine and, also, a method for the preparation of x-ray contrast agents including the above iodination step.

More particularly, a first object of the present invention is represented by a process for the preparation of 5-amino-2,4,6-triiodoisophthalic acid of formula (II) 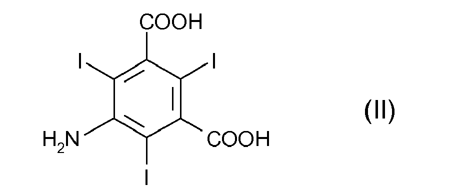 which process comprises iodinating 5-aminoisophthalic acid of formula (I) or a salt thereof 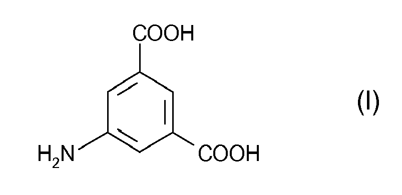 with molecular iodine in the presence of a suitable oxidizing agent.

The process of the invention is particularly advantageous as it enables the complete triiodination of the 5-aminoisophthalic acid of formula (I), or of the corresponding salt thereof, so as to lead to the corresponding 5-amino-2,4,6-triiodoisophthalic acid of formula (II), in high yields and purity.

Remarkably, and unlike previous teachings on oxidability of anilines, the above process is not affected, at least to a significant extent, by the presence of side-products deriving from the oxidative couplings occurring on the amino group. Moreover, advantageously, the process of the invention does not require the step of purification of the obtained triiodinated compound that, being isolated by filtration from the crude solution as a pure compound, fulfils the analytical specifications for the industrially produced compound.

From all of the above and, also, by efficiently consuming all of the added molecular iodine and by operating under environmentally friendly conditions, as per details below, the need of subsequent steps to recycle unreacted iodine and to treat industrial flowstreams may be minimized to a very significant extent.

As formerly reported, the iodination reaction leading to the formation of the 5-amino-2,4,6-triiodoisophthalic acid of formula (II) according to the process of the invention occurs with molecular iodine (I₂) in the presence of a suitable oxidizing agent.

To this extent, as the effective iodinating specie may be represented by iodine (I⁺) cations, at least the portion of which is first generated by molecular iodine (I₂), the unreactive iodide (I⁻) counter-ions thus produced are conveniently oxidized by the oxidizing agent, back to molecular iodine or even to iodine cations with a higher oxidation state, thus making them still available for the iodination of the aromatic ring.

Because of the above, and unless otherwise provided, suitable oxidizing agents for use in the process of the invention are those commonly employed on an industrial scale and that are capable of oxidizing iodide ions to a higher oxidation state active for iodination, for instance from (I⁰) to (I^{V}), as detailed in the following paragraphs.

Suitable examples of oxidizing agents thus include, for instance, nitric acid, sulphuric acid, iodic acid, sulphur trioxide, hydrogen peroxide, ozone, and the like.

Generally speaking, the choice of the oxidizing agent will depend from several factors among which is, for instance, their availability and, also, the selection of operating conditions enabling them to properly exert their oxidative function during the course of the reaction, so as to bring to the formation of the desired compound.

As such, and according to a first embodiment of the process of the invention, the oxidizing agent is preferably selected between hydrogen peroxide and iodic acid, the latter being even more preferred.

When molecular iodine is used in the presence of iodic acid (HIO₃), in fact, the unreactive iodide ions that are formed in the iodination reaction are converted back to molecular iodine through the so-called Dushman reaction, as per the following reaction Scheme 1

IO₃⁻ + 5I⁻ + + 6 H⁺ → 3I₂ + 3H₂O

Remarkably, this reaction further leads to a convenient reduction of the iodate (IO₃⁻) ions to molecular iodine, still available for the iodination of the aromatic ring (see, for instance, Furuichi, R. and Liebhafsky, H.A. Radioactive iodine exchange and the Dushman reaction. Bull. Chem. Soc. Japan 1973, 46, 2008-2010 and Bull. Chem. Soc. Japan 1975, 48, 745-750).

As a result, a complete triiodination of the 5-aminoisophthalic substrate is achieved so as to obtain, very advantageously, the desired compound of formula (II) in high yields and purity, by wholly consuming a stoichiometric amount of iodinating specie, that is calculated as the sum of both of the added I₂ and HIO₃, as per the following general reaction Scheme 2.

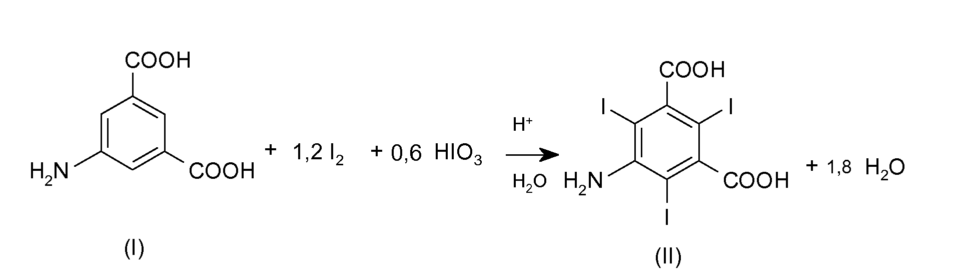

In other words, the combined use of iodine and iodic acid, as per the preferred embodiment of the invention, enables the complete triiodination of the aromatic substrate of formula (I) by avoiding, from one side, the need of any surplus of iodinating agent, especially of molecular iodine and, on the other side, the formation of by-products, especially unreactive poly-iodide ions, for instance of I₃⁻ ions, mainly deriving from the combination of I₂ with iodide ions.

In this respect, it is clear to the skilled person that the equivalent ratio between the iodinating specie considered, as said, as the sum of both of the added I₂ and HIO₃, and the 5-aminoisophtalic substrate, has to be at least equal to 3:1, as per the former general Scheme 2.

Kept safe this point, in the process of the instant invention the triiodination of the 5-aminoisophthalic substrate with iodine and iodic acid will be carried out by using at least one mole of molecular iodine for each mole of 5-aminoisophtalic substrate of formula (I). Preferably, the molar ratio between iodine and 5-aminoisophtalic substrate (I) [I₂/(I)] will vary from 1 to 1.5, more preferably from 1 to 1.3; even more preferably, the triiodination of the 5-aminoisophtalic substrate with iodine and iodic acid will be carried out by using only 1.2 moles of iodine per mole of substrate.

On the other side, because of the stoichiometry of the involved reaction, it should be clear to the skilled person that the molar ratio between iodine to iodic acid shall be at least equal to 1 : 0.5, while the molar ratio between 5-aminoisophtalic substrate (I) and iodic acid shall be at least equal to 1 : 0.6. Thus, in a preferred embodiment of the invention, the triiodination of the 5-aminoisophtalic substrate with iodine and iodic acid will be thus carried out by using a molar ratio 5-aminoisophtalic substrate : iodine : iodic acid of 1:1.2:0.6. However, we have now found that higher yields of 5-amino-2,4,6-triiodoisophthalic acid have been obtained, as reported in the experimental section, by using a slight excess of iodic acid over molecular iodine.

Therefore, and according to a further preferred embodiment of the invention, a molar ratio iodine to iodic acid ranging from 1 : 0.5 to about 1 : 1 and, more preferably, from 1 : 0.5 to about 1 : 0.8, will be employed.

To this extent, and as per the following experimental section, the proper amount of iodic acid may be added at once to the reaction mixture or, alternatively, it may be added gradually.

However, iodic acid is preferably slowly added to the reaction medium, either continuously over time or portion-wise according to conventional means, so as to minimize the occurrence of oxidized side-products.

More preferably, slow additions of iodic acid may be affected over time, for instance in a few hours, and preferably from 2 to 10 hours, as per the following experimental section.

According to another different aspect of the invention, the iodination reaction comprising the iodinating system I₂/HIO₃, as set forth above, is preferably carried out in the presence of a polar solvent and under acidic conditions. These latter are achieved in the presence of a suitable acid including, for instance, phosphoric, metanesulfonic or sulfuric acid, e.g. 96% H₂SO₄. Preferably, pH is kept between 0 and 3 and, even more preferably, between 0 and 2 by using concentrated H₂SO₄.

Interestingly, despite the fact that the above pH conditions are known to strongly deactivate any electrophilic substitution on aniline substrates, we have now found that under these same conditions, apparently unfavourable, higher amounts of 5-amino-2,4,6-triiodoisophthalic acid are unexpectedly obtained.

It should be clear to the skilled person that when operating under such acidic conditions, the aromatic substrate undergoing triiodination is represented by the 5-aminoisophthalic acid of formula (I), either employed as starting material of the process or, alternatively, formed in situ from the corresponding salt. This latter, unless otherwise provided in the present description, is preferably selected from alkali or alkali-earth metal salts of 5-aminoisophthalic acid such as, for instance, sodium, lithium, potassium, calcium or magnesium salts. Particularly preferred, among them, is the 5-aminoisophthalic acid disodium salt which can be used as such, i.e. as a pure compound or, alternatively, as comprised within a crude solution directly deriving from a previous step in the processes for the preparation of triiodinated contrast agents, for instance iopamidol.

The possible starting material of the present process are known or can be prepared according to known methods, typically including the reduction or hydrogenation of commercially available 5-nitroisophthalic acid, according to conventional means.

For a general reference to 5-aminoisophthalic acid and salts thereof, particularly the corresponding disodium salt, and their preparation see, for instance, WO 96/037458 and WO 96/037459.

As formerly reported, the above iodination reaction with I₂/HIO₃ is carried out in the presence of a polar solvent and, preferably, a protic one.

Non limiting examples of suitable solvents may thus include, for instance: water, lower alcohols C₁-C₄ and hydroalcoholic mixtures thereof, aqueous solutions including saline solutions, glycols such as, for instance, diethylene glycol, triethylene glycol, and polyethylene glycols like PEG 600, PEG1000 or PEG2000, and mixtures thereof.

In a particularly preferred embodiment of the invention, the iodination process is carried out in water or aqueous solution, that significantly contributes to reduce the costs and the environmental impact of the industrial process of the invention.

Interestingly, according to the above operating conditions, that is in the presence of an acidic aqueous environment, 5-amino-2,4,6-triiodoisophthalic acid is unexpectedly obtained in high yields and purity despite of the practical insolubility of the aromatic substrate to be iodinated.

When 5-aminoisophthalic acid is used as starting material, in fact, a proper amount of this compound is first suspended and thus maintained in the reaction medium before iodination reaction takes place. Alternatively, when using a corresponding salt of it, for instance by starting from an aqueous solution of the disodium salt, the acidic conditions are such to provoke the precipitation of the insoluble acid of formula (I) to be kept in suspension according to conventional means, e.g. under magnetic or mechanical stirring. The reaction time may vary according to the selected operative conditions and, generally, may range from about 2 to about 10 hours, more particularly from about 5 to about 8 hours.

The temperature during the process is kept constant, for instance between about 50°C to about 85°C and, preferably, from about 65°C to about 80°C, by operating according to conventional methods. Typically, by working at the formerly given temperatures, the process may reach the solvent boiling point, particularly when lower boiling solvents, like methanol, are employed. In addition, the partial sublimation of the iodine might also occur, even if the sublimed amount remains negligible if the reaction temperature is kept within the above range of values.

Nevertheless, standard cooling or condensing equipments may, for instance, be used to condensate both the solvent and the sublimate iodine that is then added to the reaction mixture according to conventional methods, for instance by adding small amounts of solvent.

Specific operative conditions and possible variations of the parameters of the process over those above indicated, for instance concerning deviations of molar ratios between reactants and substrates, time of addition of reactants, selected temperatures and the like, are all to be intended as a possible optimization of the process and, hence, are comprised within the scope of the invention. Likewise, alternative iodinating systems among those formerly reported and comprising molecular iodine in the presence of an oxidising agent other than iodic acid, for instance hydrogen peroxide, and operative conditions thereof, are also to be regarded as comprised within the scope of the invention.

Details concerning the process of the invention are however reported in the following experimental section.

According to a practical preferred embodiment of the invention, a proper amount of the 5-aminoisophthalic substrate is suspended or solubilised, as the case may be, into an aqueous solvent, typically water. The obtained solution/suspension is firstly diluted to a substrate concentration ranging from about 8 % to about 3% (w/w) and, preferably, from about 5% to about 3%, and then acidified at pH lower than 2, preferably around 1, with a suitable amount of acid, for instance with 96% H₂SO₄.

Preferably, a crude solution directly obtained from the industrial process and comprising the 5-aminoisophthalic substrate as disodium salt, at a concentration typically ranging around 7-8% (w/w), is used as starting material. This crude solution is then diluted, typically with water, to the above concentration range and then acidified at the aforementioned values, for instance with 96% H₂SO₄.

A proper amount of solid I₂ is then added to the obtained suspension that is thus kept under stirring and heated at the temperature values formerly indicated. A from pink to orange suspension is thus obtained.

Alternatively, solid I₂ may be first added to the crude solution of the 5-aminoisophthalic acid disodium salt and the obtained mixture is diluted with water and heated at about 70°-80°C, for instance for a couple of hours. The mixture is then cooled at room temperature, acidified with 96% H₂SO₄ at the formerly indicated pH value, and the obtained suspension is then heated again at the previous temperature values.

A proper amount of an aqueous solution of HIO₃ is slowly added into the reaction medium, thus causing the progressive solubilisation of the 5-aminoisophthalic substrate up to reach a homogeneous dark solution.

By proceeding with the addition of HIO₃ up to completion, the formed 5-amino-2,4,6-triiodoisophthalic acid precipitates from the reaction mixture as a pale pink solid that is filtered and dried.

The filtered compound is pure and ready to be used, without the need of any further purification, in the subsequent steps for the preparation of the desired contrast agent.

The collected filtered solution, duly acidified at the requested pH, may be optionally recycled together with a fresh amount of aromatic substrate, for a subsequent iodination step so as to convert, advantageously, any unreacted portion of 5-aminoisophthalic acid.

This recycling step may be repeated more than once, for instance two or three times, thus highly improving the global yield of the process.

Once obtained, the 5-amino-2,4,6-triiodoisophthalic acid of formula (II) may be then easily converted into iopamidol or iomeprol, by working according to known methods. The said methods comprise, essentially, the conversion of 5-amino-2,4,6-triiodoisophthalic acid into the corresponding acid dichloride, according to known methods, for instance in the presence of thionyl chloride; its subsequent condensation with the selected substrate so as to give rise to the corresponding 5-carboxamido derivative and, finally, the condensation of this latter with serinol or isoserinol and subsequent work-up including any possible cleavage of protecting groups, so to obtain the expected final compounds.

For a general reference to the preparation of iopamidol or iomeprol from 5-amino-2,4,6-triiodoisophthalic acid and operative conditions and possible variants thereof see, for instance, WO 96/037460, US 5362905, WO 97/047590 and WO 98/24757.

In addition, the process object of the present invention is of general applicability and provides, very advantageously, a route for the preparation of other iodinated contrast agents starting from the intermediate 5-amino-2,4,6-triiodoisophthalic acid.

Hence, it is a further object of the present invention a process for the preparation of the compounds of formula (III) below 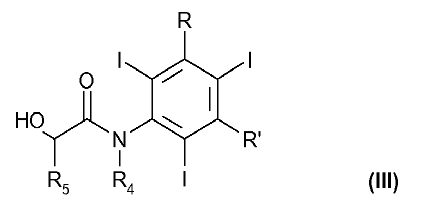 in which:
**R** and **R'** represent, the same or different from each other, a group selected from carboxy (-COOH), carboxyester (-COOR¹) and carboxamido (-CONH₂, -CONHR¹ or -CONR²R³), wherein R¹, R² and R³ are, the same or different from each other, a straight or branched C₁-C₄ alkyl group optionally substituted by one or more hydroxy groups, and
**R⁴** and **R⁵** are, the same or different from each other, hydrogen or a straight or branched C₁-C₆ alkyl group optionally substituted by one or more hydroxy or C₁-C₆ alkoxy groups, the said process comprising:
   1) preparing 5-amino-2,4,6-triiodoisophthalic acid of formula (II) 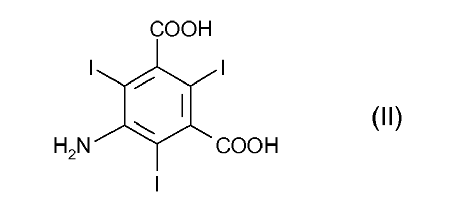 by iodinating 5-aminoisophthalic acid of formula (I) or a salt thereof 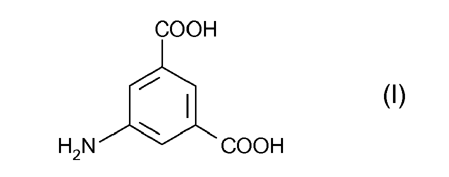 with molecular iodine in the presence of a suitable oxidizing agent; and 2) converting the compound of formula (II) in the corresponding acid dichloride and using it as an intermediate compound for the preparation of the desired compounds of formula (III).

According to the present process for preparing x-ray contrast agents, the iodination step (1) is carried out as extensively reported in the previous sections whilst, subsequent steps comprehensive of experimental operative conditions and optional variants thereof, conveniently grouped under step (2), are all to be performed according to conventional methods reported in the art.

With the aim to better illustrate the present invention, without posing any limitation to it, the following examples are now given.

### EXPERIMENTAL SECTION

### Chemical-physical characterization of the obtained compound.

The purity of the obtained 5-amino-2,4,6-triiodoisoftalic acid has been determined by HPLC using benzoic acid as internal standard

### General procedure

### HPLC chromatographic method

- Stationary phase:: Zorbax SB-Phenyl 80 Å 5 µm, 250 x 4.6 mm (Agilent
- T: echnologies)
- Mobile phase:: A: 0.015 M NaH₂PO₄ + 0.028 M H₃PO₄ B: CH₃CN
- Elution:: gradient elution gradient table:

| t (min) | phase A (%) | phase B (%) |
|---|---|---|
| 0 | 93 | 7 |
| 6 | 93 | 7 |
| 20 | 62 | 38 |
| 25 | 40 | 60 |

- Temperature:: 45 °C
- Detection:: UV (240 nm)
- Flow:: 1 mL/min
- Sample concentration:: 5 mg/mL
- Injection:: 10 µL

### Example 1

### Synthesis of 5-amino-2,4,6- triiodoisoftalic acid

In a 250 mL three naked round bottom flask equipped with thermometer, condenser and magnetic stirrer, a 3.86 % (w/w) solution of 5-aminoisophthalic acid (I) in H₂O (129.42 g; 27.6 mmol) was added and acidified at pH 1 with 96% H₂SO₄ (2 mL; 35.3 mmol) then solid I₂ (8.42 g; 33.2 mmol) was added and the mixture was heated at 72 °C by means of an oil bath. A 18.65 % (w/v) solution of HIO₃ in H₂O (20 mL; 21.2 mmol) was added over 5.2 h (adding rate 3.8 mL/h) through syringe pump; after additional 1 h at 72°C (total reaction time 6.2 h) the reaction mixture was cooled at room temperature and filtered; the solid was washed with H₂O and dried to give 5-amino-2,4,6-triiodoisophthalic acid (II) (12.74 g; 22.8 mmol) as a pale pink solid. Yield 82.6%. The product was analyzed by HPLC and, through a comparison with a standard, fulfilled the analytical specifications for 5-amino-2,4,6-triiodoisophthalic acid industrially produced.

### Example 2

In a 25 mL three naked round bottom flask equipped with thermometer, condenser and magnetic stirrer, solid I₂ (0.842 g; 3.32 mmol) was added to a 3.9 % (w/w) solution of 5-aminoisophthalic acid (I) in H₂O (13.1 g; 2.82 mmol), the mixture was heated at 75 °C in an oil bath for 1.5 h then 96% H₂SO₄ (0.46 mL; 8.11 mmol) was added by means of a dropping funnel. A 29.2 % (w/v) solution of HIO₃ in H₂O (1 mL; 1.66 mmol) was slowly added over 20 min (adding rate 3 mL/h) through a syringe pump; after 2.5 h at 75,00 °C additional 96% H₂SO₄ (0.14 mL; 2.47 mmol) was added and a 17.6 % (w/v) solution of HIO₃ in H₂O (1 mL; 1 mmol) was slowly added over 10 min (adding rate 6 mL/h). After additional 4 h at 75 °C (total reaction time 8.5 h), the reaction mixture was cooled at room temperature transferred in a separating funnel and extracted with ethyl ether (5 x 40 mL). The organic phases were collected and evaporated to give 5-amino-2,4,6-triiodoisophthalic acid (II) (1.29 g; 2.31 mmol) as pale brownish solid. Yield 81.8 %. The product was analyzed by HPLC by comparison with a standard and fulfil the analytical specifications for 5-amino-2,4,6-triiodoisophthalic acid industrially produced.

### Example 3

In a 250 mL three naked round bottom flask equipped with thermometer, condenser and magnetic stirrer, a 3.86 % (w/w) solution of 5-aminoisophthalic acid (I) in H₂O (129.42 g; 27.6 mmol) was added and acidified at pH 1 with 96% H₂SO₄ (2 mL; 35.3 mmol) then solid I₂ (5.26 g; 21.5 mmol) was added and the mixture was heated at 85 °C by means of an oil bath. A 3.08 % (w/v) solution of H₂O₂ in H₂O (25 mL; 22.6 mmol) was slowly added over 8.5 h (adding rate 3 mL/h) through a syringe pump; at the end additional solid I₂ (5.26 g; 21.5 mmol) was added. After 0.5h, 2.5h and 6h at 85 °C three portion of a 7 % (w/v) solution of H₂O₂ in H₂O (3 x 10 mL; total 61.7 mmol) was slowly added over 1.7 h each (adding rate 6 mL/h) through a syringe pump. After additional 1 h at 85 °C, the reaction mixture was cooled at room temperature and filtered; the solid was washed with H₂O and dried to give 5-amino-2,4,6-triiodoisophthalic acid (II) (12.41 g; 22.2 mmol) as pale brownish solid. Yield 80.4 %. The product was analyzed by HPLC by comparison with a standard and fulfil the analytical specifications for 5-amino-2,4,6-triiodoisophthalic acid industrially produced.

## Claims

1. A process for the preparation of 5-amino-2,4,6-triiodoisophthalic acid of formula (II) 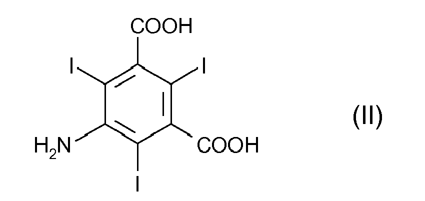 which process comprises iodinating 5-aminoisophthalic acid of formula (I) 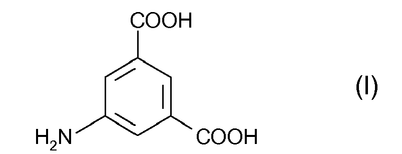 or a salt thereof with molecular iodine in the presence of an oxidizing agent.

2. A process according to claim 1 wherein the oxidizing agent is suitably selected from the group consisting of nitric acid, sulphuric acid, iodic acid, sulphur trioxide, hydrogen peroxide and ozone.

3. A process according to claim 2 wherein the oxidizing agent is iodic acid.

4. A process according to claim 3 in which the molar ratio between iodine and 5-aminoisophtalic substrate (I) is from 1 to 1,5.

5. A process according to claim 4 in which the molar ratio is from 1 to 1,3.

6. A process according to claim 3 in which the molar ratio iodine to iodic acid ranges from 1 : 0.5 to 1 : 0.8.

7. A process according to claim 3 in which the triiodination of the 5-aminoisophtalic substrate with iodine and iodic acid is carried out by using a molar ratio 5-aminoisophtalic substrate : iodine : iodic acid of 1:1.2:0.6.

8. A process according to any one of claims from 1 to 7 that is carried out in a polar solvent and in the presence of an acid selected from phosphoric, metanesulfonic or sulfuric acid.

9. A process according to claim 8 wherein the pH value of the reaction medium ranges from 0 to 3.

10. A process according to claim 8 wherein the polar solvent is selected from water, lower alcohols C₁-C₄ and hydroalcoholic mixtures thereof, aqueous saline solutions, glycols and mixtures thereof.

11. A process according to claim 10 wherein the polar solvent is selected from water, lower alcohols C₁-C₄ and hydroalcoholic mixtures thereof.

12. A process according to anyone of previous claims in which the reaction temperature is from 50°C to 85°C.

13. A process according to claim 1 comprising reacting the compound of formula (I) or the corresponding alkali or alkali-earth metal.

14. A process for the preparation of the compounds of formula (III) 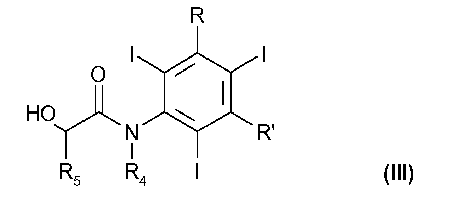 in which:
R and R' represent, the same or different from each other, a group selected from carboxy (-COOH), carboxyester (-COOR¹) and carboxamido (-CONH₂, -CONHR¹ or -CONR²R³), wherein R¹, R² and R³ are, the same or different from each other, a straight or branched C₁-C₄ alkyl group optionally substituted by one or more hydroxy groups, and
R⁴ and R⁵ are, the same or different from each other, hydrogen or a straight or branched C₁-C₆ alkyl group optionally substituted by one or more hydroxy or C₁-C₆ alkoxy groups, the said process comprising:
(1) preparing 5-amino-2,4,6-triiodoisophthalic acid of formula (II) 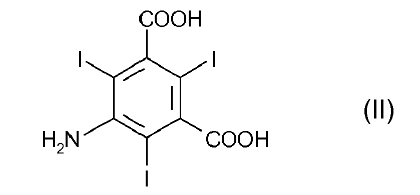 by iodinating 5-aminoisophthalic acid of formula (I) or a salt thereof 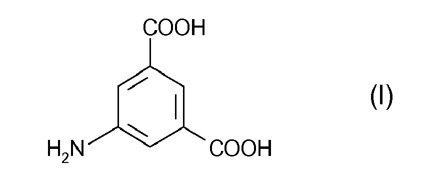 with molecular iodine in the presence of a suitable oxidizing agent; and
(2) converting the compound of formula (II) in the corresponding acid dichloride and using it as an intermediate compound for the preparation of the desired compounds of formula (III).
